(19) **Europäisches Patentamt / European Patent Office / Office européen des brevets**

(11) **EP 2 832 310 B1**

(12) **EUROPEAN PATENT SPECIFICATION**

(45) Date of publication and mention
of the grant of the patent:
**13.04.2016  Bulletin 2016/15**

(51) Int Cl.:
*A61B 18/12* (2006.01)          *H02M 7/5387* (2007.01)
*H02M 7/48* (2007.01)

(21) Application number: **14178904.0**

(22) Date of filing: **29.07.2014**

(54) **Systems for measuring tissue impedance through an electrosurgical cable**

Systeme zur Messung der Gewebeimpedanz durch ein elektrochirurgisches Kabel

Systèmes pour mesurer l'impédance d'un tissu à travers un câble électrochirurgical

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.07.2013   US 201361859601 P
29.07.2013   US 201361859624 P
14.02.2014   US 201414180965**

(43) Date of publication of application:
**04.02.2015   Bulletin 2015/06**

(73) Proprietor: **Covidien LP
Mansfield, MA 02048 (US)**

(72) Inventors:
• **Larson, Eric J.
Broomfield, CO Colorado 80020 (US)**
• **Ford, Carolyn
Louisville, CO Colorado 80027 (US)**
• **Waskiewicz, Alexander M.
Boulder, CO Colorado 80301 (US)**

(74) Representative: **Soames, Candida Jane et al
Maschio & Soames IP Limited
20 Carlton Crescent
Southampton, SO15 2ET (GB)**

(56) References cited:
**EP-A1- 2 329 782     EP-A2- 1 990 905
EP-A2- 2 025 297     US-A1- 2009 157 067**

**Description**

**BACKGROUND**

**1. Technical Field**

The present disclosure relates to electrosurgery. More particularly, the present disclosure relates to electrosurgical systems and methods for measuring tissue impedance through an electrosurgical cable.

2. **Background of Related Art**

Electrosurgery involves the application of high-frequency electric current to cut or modify biological tissue during an electrosurgical procedure. Electrosurgery is performed using an electrosurgical generator, an active electrode, and a return electrode. The electrosurgical generator (also referred to as a power supply or waveform generator) generates an alternating current (AC), which is applied to a patient's tissue through the active electrode and is returned to the electrosurgical generator through the return electrode. The alternating current typically has a frequency above 100 kilohertz (kHz) to avoid muscle and/or nerve stimulation.

During electrosurgery, the AC generated by the electrosurgical generator is conducted through tissue disposed between the active and return electrodes. The tissue's impedance converts the electrical energy (also referred to as electrosurgical energy) associated with the AC into heat, which causes the tissue temperature to rise. The electrosurgical generator controls the heating of the tissue by controlling the electric power (i.e., electrical energy per time) provided to the tissue. Although many other variables affect the total heating of the tissue, increased current density usually leads to increased heating. The electrosurgical energy is typically used for cutting, dissecting, ablating, coagulating, and/or sealing tissue.

The two basic types of electrosurgery employed are monopolar and bipolar electrosurgery. Both of these types of electrosurgery use an active electrode and a return electrode. In bipolar electrosurgery, the surgical instrument includes an active electrode and a return electrode on the same instrument or in very close proximity to one another, which cause current to flow through a small amount of tissue. In monopolar electrosurgery, the return electrode is located elsewhere on the patient's body and is typically not a part of the electrosurgical instrument itself. In monopolar electrosurgery, the return electrode is part of a device typically referred to as a return pad.

Electrosurgical generators make use of voltage and current sensors to measure quantities, such as power and tissue impedance, for controlling the output of the electrosurgical generator to achieve a desired clinical effect. The voltage and current sensors are often located inside the electrosurgical generators to save costs associated with incorporating sensors into the surgical instruments. A cable, which may be more than a meter in length, connects the electrosurgical generator to the active and return electrodes and is used to deliver electrosurgical energy to tissue being treated.

The cable creates a circuit network between the voltage and current sensors and the tissue being treated, which results in inaccurate power and impedance measurements. Thus, to more accurately measure power and impedance, many generators employ compensation algorithms that account for the impedance of the cable's circuit network. These compensation algorithms typically involve solving Kirchhoff current and voltage equations for multiple nodes in a circuit model that models the cable's circuit network. However, solutions to these equations, when implemented by a real-time embedded software system, may require a significant amount of memory and processing power.

US 2009/0157067 A1 discloses a method and apparatus for calculating current lost through a patient during a treatment of a patient using an electromagnetic energy delivery system. The system generates electromagnetic energy, contacts a skin surface of the patent, transfers the electromagnetic energy to tissue beneath the surface of the skin, detects a value of at least one characteristic of the electromagnetic energy utilizing synchronous undersampling, and calculates the current lost through the patient. The characteristic measured may be a value of current of the electromagnet energy. An adjustable matching network may be utilized to determine the electromagnetic energy delivered to the tissue of the patient. A current correction factor is determined from the impedance of the matching network and utilized to calculate the current lost through the patient.

**SUMMARY**

The electrosurgical systems and methods of the present disclosure reduce the amount of memory and processing power needed to accurately measure power and tissue impedance by using impedance equations that are based on AC filters.

In one aspect, the present disclosure features another electrosurgical generator that delivers electrosurgical energy to tissue via an electrosurgical cable and an electrosurgical instrument. The electrosurgical generator includes an output stage that generates electrosurgical energy for the electrosurgical instrument, a plurality of sensors that senses a voltage waveform and a current waveform of the generated electrosurgical energy, and a controller that controls the output stage to generate a desired level of the electrosurgical energy. The controller includes a calculator that calculates a real part of impedance based on the sensed voltage and current waveforms, an impedance compensator that estimates the impedance of tissue being treated based on the calculated real part of impedance or that corrects a target impedance curve, using a solution to a quadratic equation that models the reactive components of the electrosurgical cable, and a control signal generator that generates a control signal to control the output stage based on the estimated tissue impedance so that the estimated impedance tracks a target impedance curve or so that the calculated real part of the impedance tracks the corrected target impedance curve.

The impedance compensator may estimate the impedance of tissue or correct the target impedance curve using the solution which has frequency of generated electrosurgical energy, shunt capacitance of the cable and the real part of the impedance as input parameters.

Where the impedance calculator estimates the impedance of tissue, the generator may be configured to generate the control signal using the real part of the impedance below a predetermined resistance being reached, which represents a point when the measured real part of the impedance starts to deviate from the actual real part of the impedance of the load, and above the predetermined resistance, the estimated impedance is used.

The solution to the quadratic equation may be $R_{load} = \dfrac{1 \pm \sqrt{1 - 4 \cdot (\omega \cdot C_{cable} \cdot Re(Z))^2}}{2 \cdot Re(Z) \cdot (\omega \cdot C_{cable})^2}$, where $R_{load}$ is the resistance of the tissue, $\omega$ is the frequency of the generated electrosurgical energy, $C_{cable}$ is the shunt capacitance of a cable connecting the electrosurgical generator to an electrosurgical instrument, and $Re(Z)$ is the real part of the impedance.

The quadratic equation may be derived from a model of the cable having a series inductor and a shunt capacitor. The method may include using the larger solution to the quadratic equation as the estimate of the resistance of the tissue when a phase difference between the voltage waveform and the current waveform is less than or equal to -45 degrees, or using the smaller solution to the quadratic equation as the estimate of the resistance of the tissue when the phase difference is greater than -45 degrees.

The electrosurgical generator may include an inductor coupled to the output stage and tuned to a shunt capacitance and a series inductance of the cable so that the calculated real part of the impedance is sufficiently resistive. This hardware solution may form an independent aspect in that the above defined aspect can have the tuned inductor replacing the impedance compensator and the control signal generator may instead be defined as generating the control signal so that the calculated real part of the impedance tracks a target impedance curve. The shunt capacitance of the cable may be a capacitance value measured when electrodes of the instrument are not in contact with tissue. The inductance value of the inductor may be equal to $\dfrac{R_{load}^2 C_{cable}}{1 + \omega^2 R_{load}^2 C_{cable}^2} - L_{cable}$, where $L_{cable}$ is the series inductance of the cable. The inductance value of the inductor may be equal to

$$\frac{1}{\omega^2 C_{cable}} - L_{cable}.$$

In still another aspect, the present disclosure features a method of controlling an electrosurgical generator that delivers electrosurgical energy to tissue via an electrosurgical cable and an electrosurgical instrument coupled to the electrosurgical cable. The method includes sensing a voltage waveform and a current waveform of the generated electrosurgical energy, calculating a real part of impedance based on the sensed voltage and current waveforms, correcting a target impedance curve using a solution to a quadratic equation that models the reactive components of the electrosurgical cable, and generating a control signal to control the level of electrosurgical energy output from an output stage of the electrosurgical generator so that the calculated real part of the impedance tracks the corrected target impedance curve.

The solution to the quadratic equation may be

$$R_{load} = \frac{1 \pm \sqrt{1 - 4 \cdot (\omega \cdot C_{cable} \cdot Re(Z))^2}}{2 \cdot Re(Z) \cdot (\omega \cdot C_{cable})^2},$$

where $R_{load}$ is the resistance of the tissue, $\omega$ is the frequency of the generated electrosurgical energy, $C_{cable}$ is the shunt capacitance of a cable connecting the electrosurgical generator to an electrosurgical instrument, and $Re(Z)$ is the real part of the impedance.

The quadratic equation may be derived from a model of the cable having a series inductor and a shunt capacitor. The method may include using the larger solution to the quadratic equation as the estimate of the resistance of the tissue when a phase difference between the voltage waveform and the current waveform is less than or equal to -45 degrees, or using the smaller solution to the quadratic equation as the estimate of the resistance of the tissue when the phase difference is greater than -45 degrees.

The electrosurgical generator may include an inductor coupled to the output stage and tuned to a shunt capacitance and a series inductance of the cable so that the calculated real part of the impedance is sufficiently resistive. The shunt capacitance of the cable may be a capacitance value measured when electrodes of the instrument are not in contact with tissue. The inductance value of the inductor may be equal to $\dfrac{R_{load}^2 C_{cable}}{1 + \omega^2 R_{load}^2 C_{cable}^2} - L_{cable}$, where $L_{cable}$ is the series inductance of the cable. The inductance value of the inductor may be equal to $\dfrac{1}{\omega^2 C_{cable}} - L_{cable}$.

According to the various aspects, the electrosurgical cable creates a circuit network between the voltage and current sensors and the tissue being treated, wherein the impedance calculator or the correcting step is configured for accounting for an impedance of the circuit network.

In the various aspects, the calculator is configured for or the calculating includes determining a complex valued voltage and a complex-valued current using narrowband filters and calculating the real part of the impedance using them.

In the various aspects, cable capacitance is used as part of the solution and the controller is configured to determine the cable capacitance when active and return electrodes of the electrosurgical instrument are in an open circuit configuration, thereby setting the real load essentially to infinity.

## BRIEF DESCRIPTION OF THE DRAWINGS

Various embodiments of the present disclosure are described with reference to the accompanying drawings wherein:

FIG. 1 is an electrosurgical system in accordance with embodiments of the present disclosure;
FIG. 2 is a circuit block diagram of the electrosurgical system of FIG. 1;
FIG. 3 is a simplified circuit block diagram of the electrosurgical system of FIG. 1 including a model of an electrosurgical cable;
FIG. 4 is a graphical diagram of the real impedance of the tissue and the real part of the impedance measured at the sensors of the electrosurgical generator of FIG. 2;
FIG. 5 is a graphical diagram of the ideal impedance of the tissue and the target impedance measured at the sensors of the electrosurgical generator of FIG. 2 according to another embodiment of the present disclosure;
FIG. 6 is a graphical diagram of the ideal target real impedance at the sensors and the polynomial fit to the target real impedance at the sensors of the electrosurgical generator of FIG. 2 according to another embodiment of the present disclosure;
FIG. 7 is a simplified circuit block diagram of the electrosurgical system of FIG. 1, which includes a separate inductor according to another embodiment of the present disclosure; and
FIGS. 8 and 9 are flow diagrams of methods of controlling the output stage of the generator to compensate for the impedance of an electrosurgical cable according to embodiments of the present disclosure.

## DETAILED DESCRIPTION

As described above, the cable in an electrosurgical system creates a circuit network between the voltage and current sensors and the tissue being treated, which results in inaccurate power and impedance measurements. Thus, to more accurately measure power and impedance, many generators employ compensation algorithms that account for the impedance of the cable's circuit network. These compensation algorithms involve the measurement and storage of multiple cable parameters, such as series inductance, shunt capacitance, and resistance, which are used as constants in the solutions to the Kirchhoff current and voltage equations for multiple nodes in the model of the cable's circuit network. The compensation algorithms also involve many mathematical operations, e.g., multiplies and additions, on complex numbers having real and imaginary components.

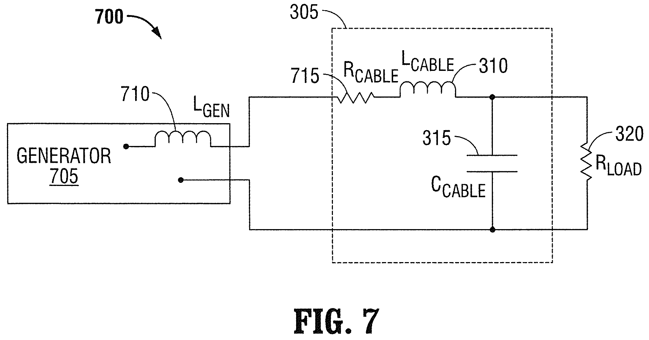

The electrosurgical systems and methods of the present disclosure reduce the amount of memory and processing power needed to accurately measure tissue impedance. The systems and methods according to the present disclosure employ a simple model of the cable for estimating the actual tissue impedance. The cable model includes an inductor and a resistance in series with the tissue being treated, and a shunt capacitor in parallel with the tissue being treated. The resistance of the cable 715 (shown in FIG. 7) is relatively small compared to the resistance of the tissue being treated and thus may be ignored. Based on sensed current and voltage waveforms, a real part of the impedance is calculated and the actual tissue impedance is estimated based on the calculated real part of the impedance.

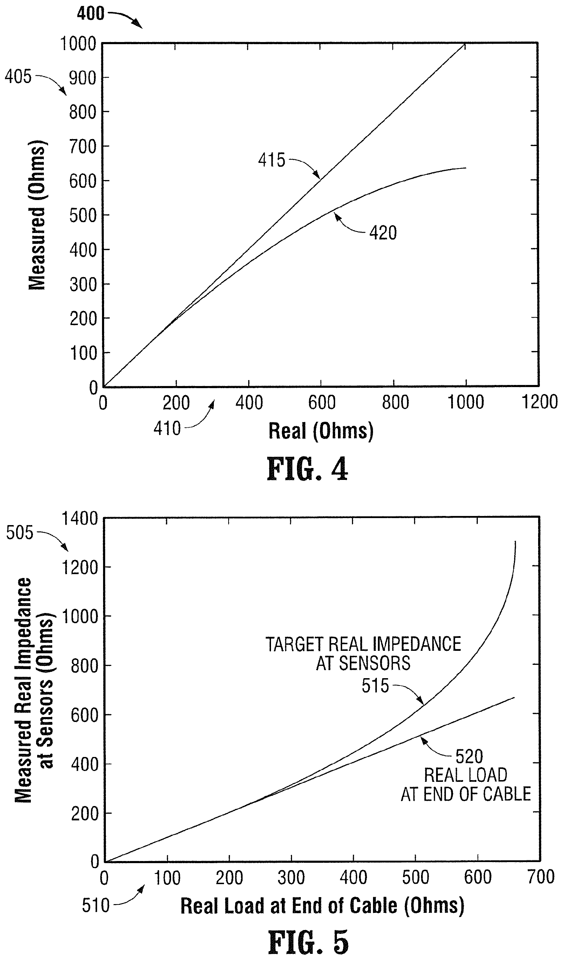

European Patent Application No. 14 156 762.8 (published as EP2799024) (and particularly paragraphs 58 to 82 and associated figures 4 to 9 of the specification as originally filed). discloses that the real part of the impedance may be obtained by determining a complex-valued voltage and a complex-valued current using narrowband filters, and calculating a real part of an impedance of the tissue using the complex-valued voltage and the complex-valued current. The real part of the tissue impedance may be calculated according to the following equation:

$$\frac{ac+bd}{c^2+d^2},$$

where $a$ is the real part of the complex-valued voltage, $b$ is the imaginary part of the complex-valued voltage, c is the real part of the complex-valued current, and d is the imaginary part of the complex-valued current. The narrowband filters may be polyphase decimator filters or Goertzel DFT filters. The polyphase decimator filters may be heterodyned carrier-centered polyphase filters having a center frequency that is a harmonic multiple of a frequency of the electrosurgical energy.

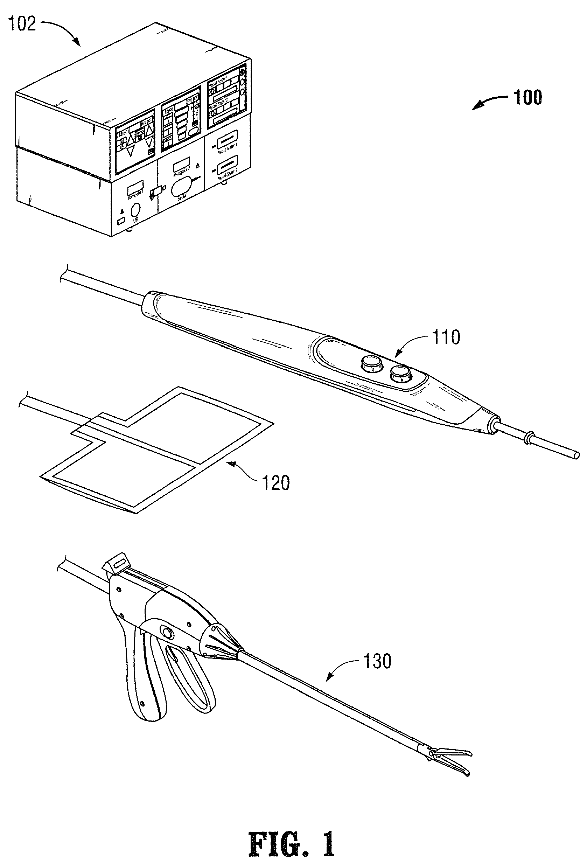

FIG. 1 illustrates an electrosurgical system 100 in accordance with embodiments of the present disclosure. The electrosurgical system 100 includes an electrosurgical generator 102 which generates electrosurgical energy to treat tissue of a patient. The electrosurgical generator 102 generates an appropriate level of electrosurgical energy based on the selected mode of operation (e.g., cutting, coagulating, ablating, or sealing) and/or the sensed voltage and current waveforms of the generated electrosurgical energy. The electrosurgical system 100 may also include a plurality of output connectors corresponding to a variety of electrosurgical instruments.

The electrosurgical system 100 further includes a monopolar electrosurgical instrument 110 having an electrode for treating tissue of the patient (e.g., an electrosurgical cutting probe or ablation electrode) with a return pad 120. The monopolar electrosurgical instrument 110 can be connected to the electrosurgical generator 102 via one of the plurality of output connectors. The electrosurgical generator 102 may generate electrosurgical energy in the form of radio frequency (RF) energy. The electrosurgical energy is supplied to the monopolar electrosurgical instrument 110, which applies the electrosurgical energy to tissue. The electrosurgical energy is returned to the electrosurgical generator 102 through the return pad 120. The return pad 120 provides sufficient contact area with the patient's tissue so as to minimize the risk of tissue damage due to the electrosurgical energy applied to the tissue.

The electrosurgical system 100 also includes a bipolar electrosurgical instrument 130. The bipolar electrosurgical instrument 130 can be connected to the electrosurgical generator 102 via one of the plurality of output connectors. The electrosurgical energy is supplied to one of the two forceps, is applied to tissue, and is returned to the electrosurgical generator 102 through the other forceps.

The electrosurgical generator 102 may be any suitable type of generator and may include a plurality of connectors to accommodate various types of electrosurgical instruments (e.g., monopolar electrosurgical instrument 110 and bipolar electrosurgical instrument 130). The electrosurgical generator 102 may also be configured to operate in a variety of modes, such as ablation, cutting, coagulation, and sealing. The electrosurgical generator 102 may include a switching mechanism (e.g., relays) to switch the supply of RF energy among the connectors to which various electrosurgical instruments may be connected. For example, when a monopolar electrosurgical instrument 110 is connected to the electrosurgical generator 102, the switching mechanism switches the supply of RF energy to the monopolar plug. In embodiments, the electrosurgical generator 102 may be configured to provide RF energy to a plurality instruments simultaneously.

The electrosurgical generator 102 includes a user interface having suitable user controls (e.g., buttons, activators, switches, or touch screens) for providing control parameters to the electrosurgical generator 102. These controls allow the user to adjust parameters of the electrosurgical energy (e.g., the power level or the shape of the output waveform) so that the electrosurgical energy is suitable for a particular surgical procedure (e.g., coagulating, ablating, tissue sealing, or cutting). The electrosurgical instruments 110 and 130 may also include a plurality of user controls. In addition, the electrosurgical generator 102 may include one or more display screens for displaying a variety of information related to the operation of the electrosurgical generator 102 (e.g., intensity settings and treatment complete indicators).

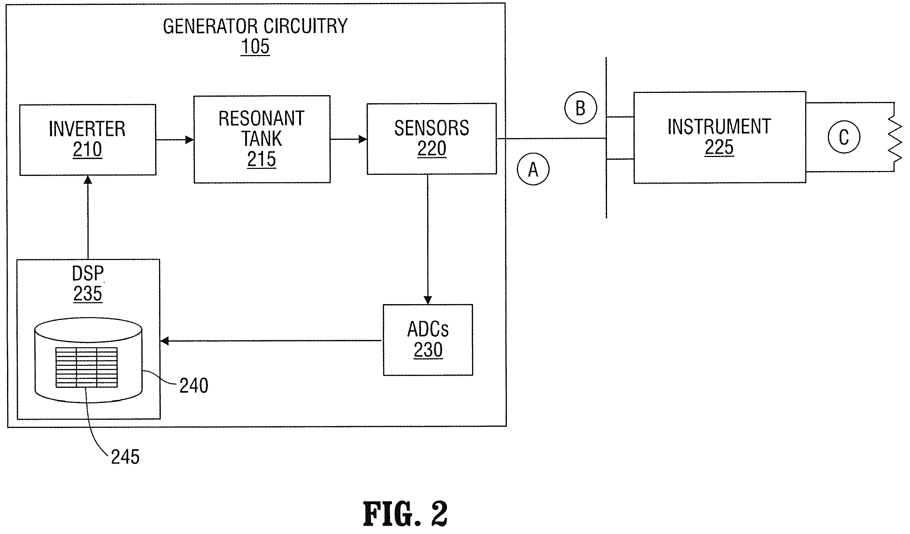

FIG. 2 is a block diagram of the electrosurgical system 100 of FIG. 1. The electrosurgical system includes generator circuitry 105, which is included in the electrosurgical generator 102 of FIG. 1, and an electrosurgical instrument 225. The generator circuitry 105 includes an inverter 210, a resonant tank circuit 215, a plurality of sensors 220, a plurality of analog-to-digital converters (ADCs) 230, and a digital signal processor 235. The generator circuitry 105 is configured to connect to an alternating current (AC) power source, such as a power outlet, which generates AC having a low frequency (e.g., 25Hz, 50 Hz, or 60 Hz). The AC power source provides AC power to the generator circuitry 105, which converts the low frequency AC to higher frequency AC that is suitable for a desired electrosurgical procedure. Specifically, the inverter 210 inverts the DC to AC. The AC waveform has a frequency suitable for an electrosurgical procedure (e.g., 472 kHz, 29.5 kHz, and 19.7 kHz).

The appropriate frequency for the electrosurgical energy may differ based on the electrosurgical procedures and modes of electrosurgery. For example, nerve and muscle stimulations cease at about 100,000 cycles per second (100 kHz) and some electrosurgical procedures can be performed safely at a radio frequency (RF) above 100 kHz. At frequencies over 100 kHz, the electrosurgical energy can pass through a patient to targeted tissue with minimal neuromuscular stimulation. For example, ablation uses a frequency of 472 kHz. Other electrosurgical procedures can be performed at frequencies lower than 100 kHz, e.g., 29.5 kHz or 19.7 kHz, with minimal risk of damaging nerves and muscles. The inverter 210 can output AC signals with various frequencies suitable for electrosurgical operations.

The resonant tank circuit 215 is coupled to the inverter 210. The resonant tank circuit 215 matches the impedance at inverter 210 to the impedance of the tissue so that there is maximum or optimal power transfer from the inverter 210 to the tissue being treated. The plurality of sensors 220 are coupled to the resonant tank circuit 215 and the electrosurgical instrument 225 to sense the voltage and current output from the generator circuitry 105 to the electrosurgical instrument 225. Point $\bigcirc$,A indicates the impedance as seen from the perspective of the generator circuitry 105. In other words, the generator circuitry 105 sees the impedance of the electrosurgical instrument 225 and the tissue being treated together at point O,A. The generator circuitry 105 is configured to compensate for the impedance in the cable disposed between points $\bigcirc$,A and $\bigcirc$,B so that the generator circuitry 105 can determine the actual impedance of the tissue at point $\bigcirc$,c.

The plurality of sensors 220 may include two or more pairs or sets of voltage and current sensors that provide redundant measurements of the voltage and current waveforms. This redundancy ensures the reliability, accuracy, and stability of the voltage and current measurements at the output of the inverter 210. In embodiments, the plurality of sensors 220 may include fewer or more sets of voltage and current sensors depending on the application or the design requirements.

The sensed voltage and current waveforms are digitally sampled by the plurality of ADCs 230 to obtain digital samples of the voltage and current waveforms sensed by the sensors 220. The plurality of ADCs 230 may sample the sensed voltage and current waveforms at a frequency that is an integer multiple of the frequency of the voltage and current generated by the electrosurgical generator 102. The sampled current and voltage waveforms are provided to the DSP 235, which includes a calculator for calculating the real part of the impedance of the tissue being treated using the sampled current and voltage waveforms, and an estimator for estimating the resistance of the tissue being treated based on the calculated real part of the tissue impedance. The DSP 235 further includes a control signal generator that generates control signals to control the output voltage and current waveforms of the inverter 210 based on the estimated resistance of the tissue. The DSP 235 includes a storage device 240 that stores instructions to implement functions for controlling the inverter 210 and information including lookup tables 245 which are used to estimate the actual impedance value of the tissue according to embodiments of the present disclosure.

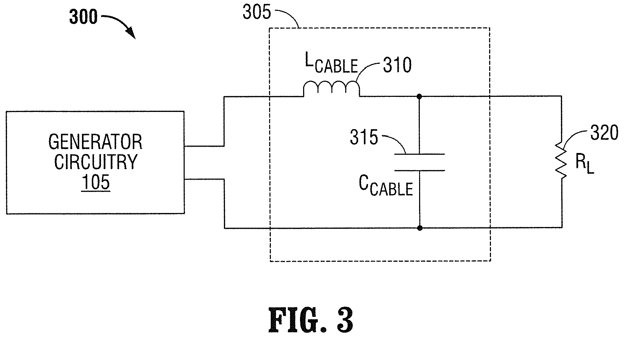

FIG. 3 is a simplified circuit block diagram illustrating an electrosurgical system 300 that includes a cable 305, which is coupled between the generator circuitry 105 and the tissue load 320. As described above, the generator circuitry 105 delivers electrosurgical energy to the tissue 320 via the cable 305. The cable 305 may be modeled as a series inductor $L_{cable}$ 310 coupled to a shunt capacitor $C_{cable}$ 315. The model may also include a series resistance, but because the resistive component is relatively small compared to the resistance of the tissue being treated, the model does not need to include the series resistance.

According to the present disclosure, the generator circuitry 105 determines the actual impedance of the load $R_L$ 320 by compensating for the reactances, i.e., the series inductance $L_{cable}$ 310 and the shunt capacitance $C_{cable}$ 315, of the cable 305. As described in FIG. 2, the impedance seen at point $\bigcirc$,A is a combination of total impedance of the cable 305 and the load 320. Theoretically, the total impedance is given by:

$$Z_{total} = \frac{V_m}{I_m} e^{-j\varphi}, \tag{1}$$

where $Z_{total}$ is the total impedance, $V_m$ is a measured voltage at the sensors 220, $I_m$ is a measured current at the sensors 220, and $\varphi$ is the phase difference between the measured voltage and measured current. The phase difference $\varphi$ is caused by the reactive components, i.e., the inductance 310 and the capacitance 315, of the cable 305.

Cable compensation is a process of determining the actual resistance $R_L$ of the load 320. Based on the cable model illustrated in FIG. 3, the total impedance is calculated as follows:

$$Z_{total} = j\omega L_C + \left( \frac{1}{j\omega C_C} // R_L \right) = j\omega L_C + \frac{R_L}{j\omega R_L C_C + 1}, \tag{2}$$

where $\omega$ is the frequency of the voltage and current. Because the total impedance is a complex value, the total impedance has a real part and an imaginary part as follows:

$$Z_{total} = \text{Re}(Z_{total}) + j\,\text{Im}(Z_{total}) = \frac{R_L}{1 + (\omega R_L C_C)^2} + j \left( \omega L_C - \frac{\omega R_L^2 C_C}{1 + (\omega R_L C_C)^2} \right). \tag{3}$$

As used herein, the total impedance $Z_{total}$ is also referred to as the impedance Z. Thus, the relationship between the calculated real part of the impedance is:

$$\text{Re}(Z) = \frac{R_L}{1 + (\omega R_L C_C)^2}. \tag{4}$$

Equation (4) can be expressed as a second order polynomial or a quadratic equation with respect to the resistance $R_L$ of the load 320, as follows:

$$(\omega^2 C_C^2 \,\text{Re}(Z))R_L^2 - R_L + \text{Re}(Z) = 0. \tag{5}$$

Equation (5) can be solved for the resistance $R_L$ of the load 320 as follows:

$$R_L = \frac{1 \pm \sqrt{1 - 4(\omega\, C_C\,\text{Re}(Z))^2}}{2\,\text{Re}(Z)(\omega\, C_C)^2}. \tag{6}$$

Based on equation (6), the actual impedance value of the load 320 can be estimated using the calculated real part of the impedance $Re(Z)$, the predetermined capacitance of the cable $C_{cable}$, and the frequency of the electrosurgical energy generated by the generator circuitry 105. The larger solution to equation (6) may be used as the estimate of the tissue resistance when a phase difference between the voltage waveform and the current waveform of the electrosurgical energy is smaller than or equal to -45 degrees and the smaller solution to equation (6) may be used as the estimate of the tissue resistance when the phase difference is greater than -45 degrees.

FIG. 4 is a graphical diagram 400 illustrating how the real part of the impedance as sensed by the generator circuitry 105 is related to the actual real part of the impedance at the load 320, i.e., the resistance of the tissue. The y-axis 405 of graph 400 represents the real part of the impedance Re(Z) in Ohms as measured at the sensors 220 of the generator circuitry 105 of FIG. 2. The x-axis 410 of graph 400 represents the resistance $R_L$ of the load 320 of FIG. 3 in Ohms. Ideally, the relationship between the real part of the impedance as measured by the generator circuitry 105 and the real load is a linear relationship as shown by curve 415. Curve 420 is the real part of the impedance $Re(Z)$ that is calculated based on measurements by the sensors 220 of the generator circuitry 105. As shown, curve 420 deviates from the ideal curve 415 as the real impedance increases. However, the two curves 415 and 420 are similar until about 250 Ohms. Thus, the generator circuitry 105 may use the measured real part of the impedance as a measurement of the real load $R_L$ when the real part of the impedance is less than or equal to 250 Ohms or another predetermined resistance. Above 250 Ohms or another predetermined resistance, the generator circuitry 105 may utilize equation (6) to estimate the real load $R_L$ based on the calculated real part of the impedance $Re(Z)$.

According to one method of the present disclosure, the DSP 235 (FIG. 2) calculates the real part of the impedance and estimates the actual real load, e.g., the tissue resistance, according to equation (6) for every measurement cycle. The DSP 235 then generates a control signal to control the inverter 210 based on the estimate of the actual real load. One of the advantages of this method is that it provides an exact solution for every measurement.

According to another method, the DSP 235 pre-populates a lookup table 245 in the DSP's storage device 240 with corrected values or correction factors. The DSP 235, e.g., a software estimator module running on the DSP 235, may then estimate the actual real load values by mapping the real part of the impedance as measured by the sensors 220 of the generator circuitry 105 to the actual real load values. When the calculated real part of the impedance is between two real part of the impedance values in the look up table, the DSP 235 selects the estimated resistance of the tissue corresponding the real part of the impedance value in the look up table that is nearest to the real part of the impedance or interpolates between the two real part of the impedance values in the look up table to determine the estimated resistance of the tissue. While the look-up table method is computationally efficient, it uses more storage and may be less accurate than the method that involves calculating the actual real load according to equation (6).

According to still another method, the DSP 235 corrects or pre-warps the target impedance curve using equation (6) and generates a control signal to control the inverter 210 of the generator circuitry 105 so that the real part of the impedance as measured by the sensors 220 tracks the corrected target impedance curve, which may be stored in a look-up table, e.g., look-up table 245. This pre-warping method is illustrated in the graphical diagram of FIG. 5, in which the y-axis represents the measured real impedance 505 at the sensors 220 and the x-axis represents the real load at the end of the cable 510. Curve 520 is the real load at the end of the cable that is achieved when the DSP 235 controls the output from the generator circuitry 105 to track the target real impedance curve 515. Like the look-up table method for estimating the real load, the pre-warping method is computationally efficient. As compared to the method for estimating the real load by calculating equation (6) every measurement cycle, the pre-warping method uses more storage and is less accurate because, like the look-up table method, the pre-warping method may require interpolation.

According to still another method, a polynomial is fit to a correction factor curve and the correction for the next target Z for each measurement is calculated using the polynomial. For example, if the center frequency is $f_c$=400kHz and the shunt capacitance of the cable $C_{cable}$ is 330 pF, then the corrected target impedance $Z_{corrected}$ could be calculated from the following second-order polynomial equation:

$$Z_{corrected} = (0.0013 * Z * Z) + (0.5221 * Z) + 26.45, \qquad (7)$$

where Z is the target impedance and coefficients of the second order polynomial equation for the corrected target impedance $Z_{corrected}$ are a function of the center frequency $f_c$ and the shunt capacitance of the cable. In some embodiments, the corrected target impedance may be calculated according to a higher-order polynomial equation if more accuracy is needed. The advantage of the polynomial fit method is that it is more computationally efficient than continually evaluating equation (6) above.

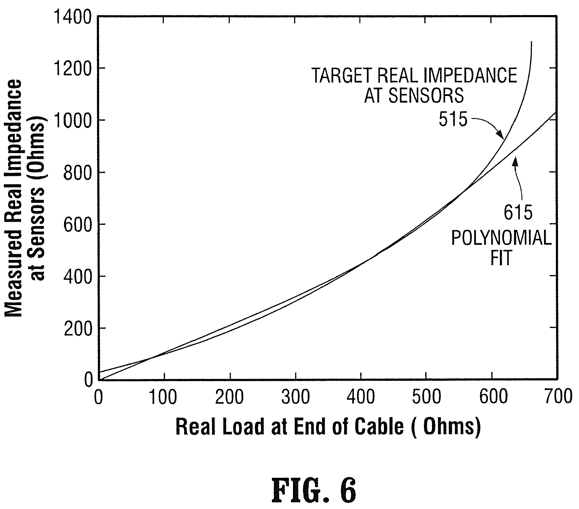

A polynomial may also be fit to the target real impedance curve 515 of FIG. 5. As shown in FIG. 6, curve 615 is a second-order polynomial fit to the target real impedance curve 515. During operation, the DSP 235 controls the inverter 210 of the generator circuitry 105 so that the real part of the impedance measured by the generator circuitry 105 tracks the polynomial fit to the target real impedance curve 615.

FIG. 7 shows a circuit block diagram of an electrosurgical system 700 that illustrates a hardware method of compensating for the cable capacitance $C_{cable}$ (315). This is achieved by adding a series inductor 710 having inductance $L_{gen}$ to the generator 705 so that:

$$L_{gen} = -\frac{1}{\omega} Im\,ag\left\{ R_{load} \,//\, \frac{1}{j\omega C_{cable}} \right\} - L_{cable}, \qquad (8)$$

where // means "in parallel with," $\omega$ is the angular frequency of the generated electrosurgical energy, $R_{load}$ is the resistance of the load 320, $C_{cable}$ is the shunt capacitance 315 of the cable 305 connected to the electrosurgical generator 705, and $L_{cable}$ is the series inductance 310 of the cable 305. After combining the resistance of the load $R_{load}$ with the impedance of the cable capacitance $\dfrac{1}{j\omega C_{cable}}$ in parallel, equation (8) becomes:

$$L_{gen} = -\frac{1}{\omega} Im\,ag\left\{ \frac{R_{load}}{j\omega R_{load} C_{cable} + 1} \right\} - L_{cable}. \qquad (9)$$

The series inductor 710 may be connected in series between the sensors 220 and the cable extending between point ○,A and point ○,B of FIG. 2 (e.g., near point ○,A) outside or inside the generator circuitry 105.

Equation (9) may be rewritten to separate out the real and imaginary parts of the parallel combination of the load resistance $R_{load}$ and the impedance of the cable capacitance $\dfrac{1}{j\omega C_{cable}}$ as follows:

$$L_{gen} = -\frac{1}{\omega} Im\,ag\left\{\frac{R_{load} - j\omega R_{load}^2 C_{cable}}{1 + \omega^2 R_{load}^2 C_{cable}^2}\right\} - L_{cable}.\qquad(10)$$

After taking the imaginary part of the parallel combination, equation (10) becomes:

$$L_{gen} = \frac{R_{load}^2 C_{cable}}{1 + \omega^2 R_{load}^2 C_{cable}^2} - L_{cable},\qquad(11)$$

Equation (11) for the inductance $L_{gen}$ can also be expressed as shown below by dividing the numerator and the denominator of the first term by $R_{load}^2$ :

$$L_{gen} = \frac{C_{cable}}{\dfrac{1}{R_{load}^2} + \omega^2 C_{cable}^2} - L_{cable}.\qquad(11\text{-}a)$$

Here, $\dfrac{1}{R_{load}^2}$ is much smaller than $\omega^2 C_{cable}^2$ and thus is negligible. The inductance $L_{gen}$ may then be expressed as follows:

$$L_{gen} \cong \frac{C_{cable}}{\omega^2 C_{cable}^2} - L_{cable} = \frac{1}{\omega^2 C_{cable}} - L_{cable},\qquad(11\text{-}b)$$

As shown, equation (11-b) for the inductance $L_{gen}$ is independent of the resistance of the load $R_{load}$ (i.e., the resistance of the tissue). Thus, the cable capacitance $C_{cable}$ (315) may be compensated for by adding a series inductor 710 having inductance $L_{gen}$ determined according to equation (11-b) to the generator 705.

Thus, the total impedance $Z_{total}$ as seen by the sensors 220 (see FIG. 2) at the generator 705 becomes:

$$Z_{total} = j\omega(L_{gen} + L_{cable}) + R_{cable} + R_{load} \mathbin{/\!/} \frac{1}{j\omega C_{cable}}.\qquad(12)$$

Substituting equation (8) for the series inductance $L_{gen}$ of equation (12) results in the following equation:

$$Z_{total} = j\omega\left\{-\frac{1}{\omega} Im\,ag\left\{R_{load} \mathbin{/\!/} \frac{1}{j\omega C_{cable}}\right\} - L_{cable} + L_{cable}\right\} + R_{cable} +$$
$$Re\left\{R_{load} \mathbin{/\!/} \frac{1}{j\omega C_{cable}}\right\} + j\,Im\,ag\left\{R_{load} \mathbin{/\!/} \frac{1}{j\omega C_{cable}}\right\}.\qquad(13)$$

Since the inductance of the cable $L_{cable}$ is subtracted out of equation (13), equation (13) may be rewritten as follows:

$$Z_{total} = -j\,Im\,ag\left\{R_{load} \mathbin{/\!/} \frac{1}{j\omega C_{cable}}\right\} + R_{cable} + Re\left\{R_{load} \mathbin{/\!/} \frac{1}{j\omega C_{cable}}\right\} +$$
$$j\,Im\,ag\left\{R_{load} \mathbin{/\!/} \frac{1}{j\omega C_{cable}}\right\}. \tag{14}$$

Further, as shown in equation (14), the imaginary part of the parallel combination is subtracted out. Thus, equation (14) becomes:

$$Z_{total} = R_{cable} + Re\left\{R_{load} \mathbin{/\!/} \frac{1}{j\omega C_{cable}}\right\}. \tag{15}$$

After combining the resistance of the load $R_{load}$ with the impedance of the cable capacitance $\dfrac{1}{j\omega C_{cable}}$ in parallel, equation (15) becomes:

$$Z_{total} = R_{cable} + Re\left\{\frac{R_{load}}{j\omega R_{load} C_{cable} + 1}\right\}. \tag{16}$$

Equation (16) may be rewritten to separate out the real and imaginary parts of the parallel combination of the load resistance $R_{load}$ and the impedance of the cable capacitance $\dfrac{1}{j\omega C_{cable}}$ as follows:

$$Z_{total} = R_{cable} + Re\left\{\frac{R_{load} - j\omega R_{load}^2 C_{cable}}{1 + \omega^2 R_{load}^2 C_{cable}^2}\right\}. \tag{17}$$

After taking the real part of the parallel combination, equation (17) becomes:

$$Z_{total} = R_{cable} + \frac{R_{load}}{1 + \omega^2 R_{load}^2 C_{cable}^2}. \tag{18}$$

Equation (18) indicates that if the inductance $L_{gen}$ of the series inductor 710 is tuned properly, the load presented to the generator circuitry 105 is purely resistive. The advantage of hardware compensation is that software compensation would not be needed if the inductance $L_{gen}$ is properly tuned. Even if the inductance $L_{gen}$ is not properly tuned, the added series inductor 710 would reduce the effects of the shunt capacitance in the cable. The software compensation methods described above may be used in combination with the series inductor 710 to further reduce the effects of the shunt capacitance in the cable. For hardware compensation, the series inductor 710 would need to be tuned for each of the different cables that are used. The series inductor 710 may be placed after the sensors and in series with the cable and the load. The series inductor 710 may alternatively be placed next to the output of the electrosurgical generator.

The cable capacitance C can be determined by turning on the generator with the jaws of the instrument open, that is, setting the real load to, essentially, infinity. Then, the open circuit version of FIG. 3 reduces to:

$$|Z| = \omega C \tag{19}$$

Thus, when |Z| is measured at the sensors 220 of the generator circuitry 105, the capacitance C can be determined.

The systems and methods of measuring tissue impedance described above may be employed in a variety of tissue treatment algorithms including a tissue treatment algorithm having a pre-heating phase and an impedance control phase. At the start of the pre-heating phase, the level of current generated by the generator and supplied to the tissue is low and the impedance of the tissue starts at an initial impedance value. During the pre-heating phase, the level of current supplied to the tissue is increased or ramped upward at a predetermined rate so that the temperature of the tissue increases and the tissue impedance decreases. The ramping of the current continues until (1) the maximum allowable current value is reached, or (2) there is a "tissue reaction." The term "tissue reaction" refers to a point at which intracellular and/or extra-cellular fluid begins to boil and/or vaporize, resulting in an increase in tissue impedance. In the case when the maximum allowable current value is reached, the maximum current value is maintained until the tissue reacts.

When the tissue reacts, the tissue treatment algorithm transitions to the impedance control phase. In the impedance control phase, the tissue treatment algorithm first calculates a target tissue impedance curve or trajectory and a target rate of change of tissue impedance (dZ/dt). Then, the tissue treatment algorithm controls the power level of the electrosurgical energy output from the generator so that the measured tissue impedance as measured according to the systems and methods of the present disclosure tracks the target tissue impedance trajectory and the target rate of change of tissue impedance.

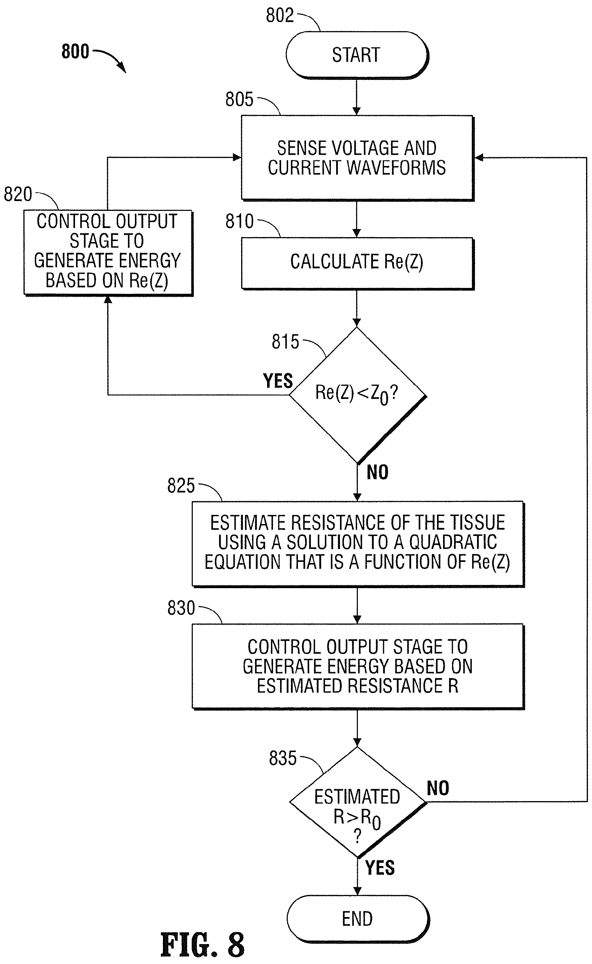

FIG. 8 illustrates a method 800 of controlling the output stage of the generator to compensate for parasitics in the electrosurgical cable during the impedance control phase. This method may be implemented in the DSP 235, which may be replaced by any suitable type of processor, of the electrosurgical generator 102 of FIG. 2. After the impedance control phase starts in step 802, the method 800 first involves sensing a voltage waveform and a current waveform of the generated electrosurgical energy in step 805. In step 810, a real part of an impedance is calculated based upon the sensed voltage and current waveforms. In step 815, it is determined whether the calculated impedance $Re(Z)$ is less than a predetermined impedance value $Z_0$. The predetermined impedance value $Z_0$ represents an impedance threshold value near which the calculated real part of the impedance $Re(Z)$ starts to deviate from the actual resistance of the tissue load.

If, in step 815, it is determined that the calculated real part of the impedance is less than the predetermined impedance value, then the output stage is controlled in step 820 to generate electrosurgical energy based on the calculated real part of the impedance. Then, the method 800 returns to step 805 to repeat the control process. If, in step 815, it is determined that the calculated real part of the impedance is not less than the predetermined impedance value, then, in step 825, a resistance of the tissue is estimated using a solution to a quadratic equation that is a function of the calculated real part of the impedance. Then, in step 830, a control signal for controlling the output stage is generated based on the estimated resistance of the tissue.

Next, in step 835, it is determined whether the estimated resistance of the tissue is greater than a predetermined tissue resistance $R_0$. If the estimated resistance of the tissue is greater than the predetermined tissue resistance $R_0$, then the method ends. Otherwise, the method 800 returns to step 805 to repeat the method 800.

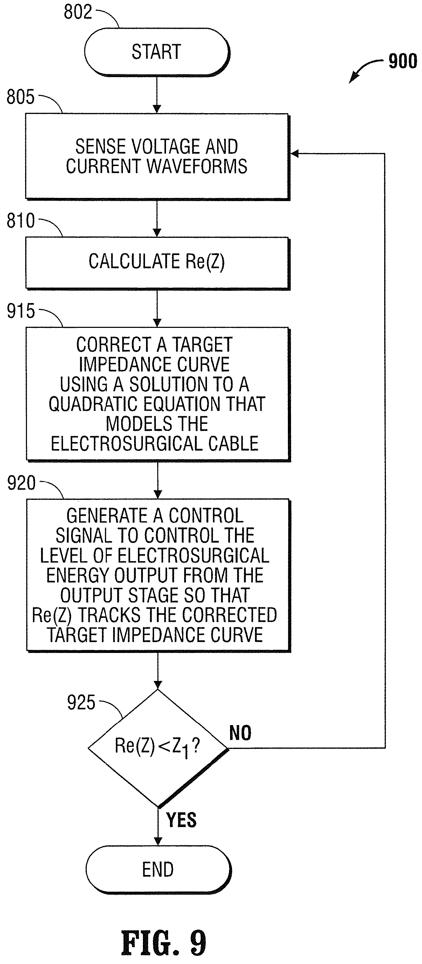

FIG. 9 illustrates another method 900 of controlling the output stage of the generator to compensate for parasitics in the electrosurgical cable. As in FIG. 8, after the impedance control algorithm starts in step 802, the voltage and current waveforms are sensed at the generator in step 805 and the real part of the impedance is calculated in step 810. Next, in step 915, the target impedance curve is corrected using a solution to a quadratic equation that models the electrosurgical cable as described above with respect to FIG. 6. Then, in step 920, a control signal is generated to control the level of electrosurgical energy output from the output stage so that the real part of the impedance tracks the corrected target impedance curve.

Next, in step 925, it is determined whether the real part of the impedance $Re(Z)$ is greater than a predetermined impedance $Z_1$. If the real part of the impedance is greater than the predetermined tissue resistance $Z_1$, then the method ends. Otherwise, the method 900 returns to step 805 to repeat the method 900.

Although the illustrative embodiments of the present disclosure have been described herein with reference to the accompanying drawings, it is to be understood that the disclosure is not limited to those precise embodiments, and that various other changes and modification may be effected therein by one skilled in the art without departing from the scope of the disclosure.

**Claims**

1. An electrosurgical generator (102) that delivers electrosurgical energy to tissue via an electrosurgical cable (305) and an electrosurgical instrument (225), the electrosurgical generator comprising:

an output stage coupled to an electrical energy source and configured to generate electrosurgical energy to the electrosurgical instrument;
a plurality of sensors (220) configured to sense a voltage waveform and a current waveform of the generated electrosurgical energy; and
a controller (235) configured to control the output stage to generate a desired level of the electrosurgical energy, **characterized in that** the controller comprises:

a calculator configured to calculate a real part of impedance based on the sensed voltage and current waveforms;
an impedance compensator configured to correct a target impedance curve using a solution to a quadratic equation that models the reactive components of the electrosurgical cable; and
a control signal generator configured to generate a control signal to control the output stage so that the calculated real part of the impedance tracks the corrected target impedance curve.

2. The electrosurgical generator according to claim 1, wherein the solution to the quadratic equation is

$$R_{load} = \frac{1 \pm \sqrt{1 - 4 \cdot ( \omega \cdot C_{cable} \cdot Re( Z ))^2}}{2 \cdot Re( Z ) \cdot ( \omega \cdot C_{cable} )^2}$$ , where $R_{load}$ is the resistance of the tissue, $\omega$ is the frequency of the generated electrosurgical energy, $C_{cable}$ is the shunt capacitance of a cable connecting the electrosurgical generator to an electrosurgical instrument, and $Re(Z)$ is the real part of the impedance.

3. The electrosurgical generator according to claim 2, wherein the resistance of the tissue is the larger solution to the quadratic equation when a phase difference between the voltage waveform and the current waveform is less than or equal to -45 degrees, or the smaller solution when the phase difference is greater than -45 degrees.

4. The electrosurgical generator according to claim 1, 2 or 3, wherein the model of the reactive components of the electrosurgical cable includes an inductor (310) in series with the tissue and a shunt capacitor (315) in parallel with the tissue.

5. The electrosurgical generator according to claim 2, 3 or 4, wherein the shunt capacitance of the cable is a capacitance value measured when electrodes of the instrument are not in contact with tissue.

6. The electrosurgical generator according to any one of claims 1 to 5, further comprising an inductor (710) coupled to the output stage and tuned to a shunt capacitance and a series inductance of the cable so that the calculated real part of the impedance is sufficiently resistive.

7. The electrosurgical generator according to claim 6, wherein the shunt capacitance of the cable is a capacitance value measured when electrodes of the instrument are not in contact with tissue.

8. The electrosurgical generator according to claim 6, wherein an inductance value of the inductor is equal

$$\frac{R_{load}^2 C_{cable}}{1 + \omega^2 R_{load}^2 C_{cable}^2} - L_{cable}$$ , where $\omega$ is the frequency of the generated electrosurgical energy, $C_{cable}$ is the shunt capacitance of a cable connecting the electrosurgical generator to an electrosurgical instrument, $R_{load}$ is the resistance of the tissue, and $L_{cable}$ is the series inductance of the cable.

9. The electrosurgical generator according to claim 6, wherein an inductance value of the inductor is equal to

$$\frac{1}{\omega^2 C_{cable}} - L_{cable}$$ , where $\omega$ is the frequency of the generated electrosurgical energy, $C_{cable}$ is the shunt capacitance of a cable connecting the electrosurgical generator to an electrosurgical instrument, and $L_{cable}$ is the series inductance of the cable.

10. A non-transitory computer-readable storage medium storing a program that, when executed by a processor, performs a method of controlling an electrosurgical generator according to claim 1 that delivers electrosurgical energy to tissue via an electrosurgical cable and an electrosurgical instrument, the method comprising:

sensing a voltage waveform and a current waveform of the generated electrosurgical energy;
**characterized by** further comprising:

calculating a real part of impedance based on the sensed voltage and current waveforms;
correcting a target impedance curve using a solution to a quadratic equation that models the reactive components of the electrosurgical cable; and
generating a control signal to control the output from an output stage of the electrosurgical generator so that the calculated real part of the impedance tracks the corrected target impedance curve.

**Patentansprüche**

1. Elektrochirurgischer Generator (102), der elektrochirurgische Energie über ein elektrochirurgisches Kabel (305) und ein elektrochirurgisches Instrument (225) an Gewebe abgibt, wobei der elektrochirurgische Generator umfasst:

eine Ausgangsstufe, die an eine elektrische Energiequelle gekoppelt ist und dafür konfiguriert ist, elektrochirurgische Energie für das elektrochirurgische Instrument zu erzeugen;
eine Mehrzahl von Sensoren (220), die dafür konfiguriert sind, eine Spannungswellenform und eine Stromwellenform der erzeugten elektrochirurgischen Energie zu erfassen; und
eine Steuerung (325), die dafür konfiguriert ist, die Ausgangsstufe zu steuern, um ein erwünschtes Ausmaß der elektrochirurgischen Energie zu erzeugen,
**dadurch gekennzeichnet, dass** die Steuerung umfasst:

einen Rechner, der dafür konfiguriert ist, einen Realteil der Impedanz auf der Grundlage der erfassten Spannungs- und Stromwellenform zu berechnen;
einen Impedanzkompensator, der dafür konfiguriert ist, eine Zielimpedanzkurve mittels einer Lösung für eine quadratische Gleichung zu korrigieren, die die reaktiven Komponenten des elektrochirurgischen Kabels abbildet; und
einen Steuersignalgenerator, der dafür konfiguriert ist, ein Steuersignal zum Steuern der Ausgangsstufe zu erzeugen, sodass der berechnete Realteil der Impedanz der korrigierten Zielimpedanzkurve folgt.

2. Elektrochirurgischer Generator nach Anspruch 1, wobei die Lösung für die quadratische Gleichung

$$R_{load} = \frac{1 \pm \sqrt{1 - 4 \cdot (\omega \cdot C_{cable} \cdot Re(Z))^2}}{2 \cdot Re(Z) \cdot (\omega \cdot C_{cable})^2}$$ ist, wobei $R_{load}$ der Widerstand des Gewebes ist, $\omega$ die Frequenz der erzeugten elektrochirurgischen Energie ist, $C_{cable}$ die Parallelkapazität eines Kabels ist, das den elektrochirurgischen Generator mit einem elektrochirurgischen Instrument verbindet, und $Re(Z)$ der Realteil der Impedanz ist.

3. Elektrochirurgischer Generator nach Anspruch 2, wobei der Widerstand des Gewebes die größere Lösung für die quadratische Gleichung ist, wenn eine Phasendifferenz zwischen der Spannungswellenform und der Stromwellenform weniger als oder gleich -45 Grad ist, oder die kleinere Lösung, wenn die Phasendifferenz größer als -45 Grad ist.

4. Elektrochirurgischer Generator nach Anspruch 1, 2 oder 3, wobei das Modell der reaktiven Komponenten des elektrochirurgischen Kabels einen Induktor (310) in Reihe mit dem Gewebe und einen Parallelkondensator (315) parallel zum Gewebe aufweist.

5. Elektrochirurgischer Generator nach Anspruch 2, 3 oder 4, wobei die Parallelkapazität des Kabels ein Kapazitätswert ist, der gemessen wird, wenn Elektroden des Instruments nicht mit Gewebe in Kontakt stehen.

6. Elektrochirurgischer Generator nach einem der Ansprüche 1 bis 5, des Weiteren umfassend einen Induktor (710), der an die Ausgangsstufe gekoppelt ist und auf eine Parallelkapazität und eine Reiheninduktanz des Kabels eingestellt ist, sodass der berechnete Realteil der Impedanz ausreichend resistiv ist.

7. Elektrochirurgischer Generator nach Anspruch 6, wobei die Parallelkapazität des Kabels ein Kapazitätswert ist, der gemessen wird, wenn Elektroden des Instruments nicht mit Gewebe in Kontakt stehen.

13

**8.** Elektrochirurgischer Generator nach Anspruch 6, wobei ein

Induktanzwert des Induktors gleich $\dfrac{R_{load}^2 C_{cable}}{1 + \omega^2 R_{load}^2 C_{cable}^2} - L_{cable}$ ist, wobei $\omega$ die Frequenz der erzeugten elektrochirurgischen Energie ist, $C_{cable}$ die Parallelkapazität eines Kabels ist, das den elektrochirurgischen Generator mit einem elektrochirurgischen Instrument verbindet, $R_{load}$ der Widerstand des Gewebes ist und $L_{cable}$ die Reiheninduktanz des Kabels ist.

**9.** Elektrochirurgischer Generator nach Anspruch 6, wobei ein

Induktanzwert des Induktors gleich $\dfrac{1}{\omega^2 C_{cable}} - L_{cable}$ ist, wobei $\omega$ die Frequenz der erzeugten elektrochirurgischen Energie ist, $C_{cable}$ die Parallelkapazität eines Kabels ist, das den elektrochirurgischen Generator mit einem elektrochirurgischen Instrument verbindet, und $L_{cable}$ die Reiheninduktanz des Kabels ist.

**10.** Nichtflüchtiges computerlesbares Speichermedium, auf dem ein Programm gespeichert ist, das beim Ausführen durch einen Prozessor ein Verfahren zum Steuern eines elektrochirurgischen Generators nach Anspruch 1 durchführt, der elektrochirurgische Energie über ein elektrochirurgisches Kabel und ein elektrochirurgisches Instrument an Gewebe abgibt, wobei das Verfahren umfasst:

Erfassen einer Spannungswellenform und einer Stromwellenform der erzeugten elektrochirurgischen Energie; **dadurch gekennzeichnet, dass** es des Weiteren umfasst:

Berechnen eines Realteils der Impedanz auf der Grundlage der erfassten Spannungs- und Stromwellenform;
Korrigieren einer Zielimpedanzkurve mittels einer Lösung für eine quadratische Gleichung, die die reaktiven Komponenten des elektrochirurgischen Kabels abbildet; und
Erzeugen eines Steuersignals zum Steuern des Ausgangs einer Ausgangsstufe des elektrochirurgischen Generators, sodass der berechnete Realteil der Impedanz der korrigierten Zielimpedanzkurve folgt.

## Revendications

**1.** Générateur électrochirurgical (102) qui délivre de l'énergie électrochirurgicale à un tissu via un câble électrochirurgical (305) et un instrument électrochirurgical (225), le générateur électrochirurgical comprenant :

un étage de sortie couplé à une source d'énergie électrique et configuré pour produire de l'énergie électrochirurgicale pour l'instrument électrochirurgical ;
une pluralité de capteurs (220) configurés pour détecter une forme d'onde de tension et une forme d'onde de courant de l'énergie électrochirurgicale produite ; et
une unité de commande (235) configurée pour commander l'étage de sortie pour produire un niveau souhaité de l'énergie électrochirurgicale, **caractérisé en ce que** l'unité de commande comprend :

un calculateur configuré pour calculer une partie réelle d'impédance basée sur les formes d'onde de tension et de courant détectées ;
un compensateur d'impédance configuré pour corriger une courbe d'impédance cible en utilisant une solution d'une équation quadratique qui modélise les composants réactifs du câble électrochirurgical ; et
un générateur de signal de commande configuré pour produire un signal de commande pour commander l'étage de sortie de sorte que la partie réelle calculée de l'impédance suit la courbe d'impédance cible corrigée.

**2.** Générateur électrochirurgical selon la revendication 1, dans lequel la solution de l'équation quadratique est

$$R_{load} = \frac{1 \pm \sqrt{1 - 4 \cdot (\omega \cdot C_{cable} \cdot Re(Z))^2}}{2 \cdot Re(Z) \cdot (\omega \cdot C_{cable})^2} \ ,$$

où R$_{load}$ est la résistance du tissu, ω est la fréquence de l'énergie électrochirurgicale produite, C$_{cable}$ est la capacité de shunt d'un câble reliant le générateur électrochirurgical à un instrument électrochirurgical et *Re(Z)* est la partie réelle de l'impédance.

3. Générateur électrochirurgical selon la revendication 2, dans lequel la résistance du tissu est la solution plus grande de l'équation quadratique quand une différence de phase entre la forme d'onde de tension et la forme d'onde de courant est inférieure ou égale à -45 degrés, ou la solution plus petite quand la différence de phase est plus grande que -45 degrés.

4. Générateur électrochirurgical selon la revendication 1, 2 ou 3, dans lequel le modèle des composants réactifs du câble électrochirurgical inclut une bobine d'inductance (310) en série avec le tissu et un condensateur de shunt (315) en parallèle avec le tissu.

5. Générateur électrochirurgical selon la revendication 2, 3 ou 4, dans lequel la capacité de shunt du câble est une valeur de capacité mesurée quand des électrodes de l'instrument ne sont pas en contact avec un tissu.

6. Générateur électrochirurgical selon n'importe laquelle des revendications 1 à 5, comprenant en outre une bobine d'inductance (710) couplée à l'étage de sortie et accordée sur une capacité de shunt et une inductance en série du câble de sorte que la partie réelle calculée de l'impédance est suffisamment résistive.

7. Générateur électrochirurgical selon la revendication 6, dans lequel la capacité de shunt du câble est une valeur de capacité mesurée lorsque les électrodes de l'instrument ne sont pas en contact avec un tissu.

8. Générateur électrochirurgical selon la revendication 6, dans lequel une valeur d'inductance de la bobine d'inductance est égale à

$$\frac{R_{load}^2 C_{cable}}{1 + \omega^2 R_{load}^2 C_{cable}^2} - L_{cable},$$

où ω est la fréquence de l'énergie électrochirurgicale produite, C$_{cable}$ est la capacité de shunt d'un câble reliant le générateur électrochirurgical à un instrument électrochirurgical, R$_{load}$ est la résistance du tissu et L$_{cable}$ est l'inductance en série du câble.

9. Générateur électrochirurgical selon la revendication 6, dans lequel une valeur d'inductance de la bobine d'inductance est égale à

$$\frac{1}{\omega^2 C_{cable}} - L_{cable},$$

où ω est la fréquence de l'énergie électrochirurgicale produite, C$_{cable}$ est la capacité de shunt d'un câble reliant le générateur électrochirurgical à un instrument électrochirurgical et L$_{cable}$ est l'inductance en série du câble.

10. Support de stockage non transitoire lisible par ordinateur stockant un programme qui, lorsqu'il est exécuté par un processeur, effectue un procédé de commande d'un générateur électrochirurgical selon la revendication 1 qui délivre de l'énergie électrochirurgicale à un tissu via un câble électrochirurgical et un instrument électrochirurgical, le procédé comprenant :

la détection d'une forme d'onde de tension et d'une forme d'onde de courant de l'énergie électrochirurgicale produite ;

caractérisé comme comprenant en outre :

le calcul d'une partie réelle d'impédance basée sur les formes d'onde de tension et de courant détectées ;
la correction d'une courbe d'impédance cible en utilisant une solution à une équation quadratique qui modélise les composants réactifs du câble électrochirurgical ; et
la production d'un signal de commande pour commander la sortie provenant d'un étage de sortie du générateur électrochirurgical de sorte que la partie réelle calculée de l'impédance suit la courbe d'impédance cible corrigée.

**FIG. 1**

**FIG. 2**

**300**

305

$L_{CABLE}$ ~310

315

$C_{CABLE}$

320

$R_L$

GENERATOR CIRCUITRY 105

# FIG. 3

**FIG. 4**

**FIG. 5**

**FIG. 6**

**FIG. 7**

**800**

802
START

805
SENSE VOLTAGE AND
CURRENT WAVEFORMS

820
CONTROL OUTPUT
STAGE TO
GENERATE ENERGY
BASED ON Re(Z)

810
CALCULATE Re(Z)

815
YES
$Re(Z) < Z_0$?
NO

825
ESTIMATE RESISTANCE OF THE TISSUE
USING A SOLUTION TO A QUADRATIC
EQUATION THAT IS A FUNCTION OF Re(Z)

830
CONTROL OUTPUT STAGE TO
GENERATE ENERGY BASED ON
ESTIMATED RESISTANCE R

835
NO
ESTIMATED
$R > R_0$
?
YES

END

**FIG. 8**

**802** START

**900**

**805** SENSE VOLTAGE AND CURRENT WAVEFORMS

**810** CALCULATE Re(Z)

**915** CORRECT A TARGET IMPEDANCE CURVE USING A SOLUTION TO A QUADRATIC EQUATION THAT MODELS THE ELECTROSURGICAL CABLE

**920** GENERATE A CONTROL SIGNAL TO CONTROL THE LEVEL OF ELECTROSURGICAL ENERGY OUTPUT FROM THE OUTPUT STAGE SO THAT Re(Z) TRACKS THE CORRECTED TARGET IMPEDANCE CURVE

**925** $Re(Z) < Z_1$? NO

YES

END

# FIG. 9

**EP 2 832 310 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20090157067 A1
- EP 14156762 A
- EP 2799024 A